# EUROPÄISCHE PATENTSCHRIFT

(11) **EP 0 824 016 B1**
(45) Veröffentlichungstag und Bekanntmachung des Hinweises auf die Patenterteilung: **26.01.2000**
(21) Anmeldenummer: 96202282.8
(22) Anmeldetag: 14.08.1996
(51) Int. Cl.: A61F 13/00, A61F 5/34

(54) **Vorrichtung zur Behandlung von Narbengewebe**
Apparatus for the treatment of scar tissue
Dispositif de traitement de tissus cicatriciels

(43) Veröffentlichungstag der Anmeldung: 18.02.1998
(73) Patentinhaber: Otto Bock Orthopädische Industrie Besitz- und Verwaltungs-Kommanditgesellschaft, 37115 Duderstadt (DE); K.U. LEUVEN RESEARCH & DEVELOPMENT, 3000 Leuven (BE)
(72) Erfinder: De Cubber, Jan Prosper Dennis, 1930 Zaventem (BE); Van den Kerckhove, Eric André Maria Clement, 3001 Heverlee (BE); Boeckx, Willy Dennis, 3210 Linden (BE)
(74) Vertreter: Gramm, Werner, Prof. Dipl.-Ing.

(56) Entgegenhaltungen:
- EP-A- 0 035 583
- WO-A-91/18571
- US-A- 4 193 401
- US-A- 4 202 331
- US-A- 4 224 945
- US-A- 4 436 089
- US-A- 5 376 067

## Beschreibung

Die Erfindung betrifft eine Vorrichtung zur Behandlung von Narbengewebe, zu dessen Abdeckung die Vorrichtung einen Abdeckkörper umfaßt, der zumindest ein mit einem Druckmedium befüllbares Element (Druckelement) zur Druckbeaufschlagung des Narbengewebes aufweist und von einem dem Druckelement als Widerlager dienenden Trageteil übergriffen ist, das mit Befestigungsmitteln am Körper des Patienten festlegbar ist.

Eine derartige Vorrichtung wird angewendet, um der Bildung hypertrophen Narbengewebes bei durch Verbrennen entstandenen Verletzungen der Oberhaut und Lederhaut entgegenzuwirken.

Hypertrophe Narben entstehen im allgemeinen, wenn die Haut vollständig - das heißt sowohl die Oberhaut als auch die darunterliegende Lederhaut - verletzt ist, wie beispielsweise bei Verbrennungen zweiten oder dritten Grades. Diese Narben sind nicht nur verunstaltend, besonders wenn sie sich an gut sichtbaren Körperstellen wie dem Gesicht oder dem Hals befinden, sondern sie können auch zu Körperfunktionsstörungen führen, wenn sie sich in der Nähe von Muskeln oder Gelenken bilden. Zu denken wäre in diesem Zusammenhang an die Verbrennung der Handfläche, bei der diese Narben zu einer permanenten Handgelenkkontraktur führen können. Verbrennungsbedingte Narben verursachen beim Patienten somit sowohl körperliche als auch seelische Schäden, und der Genesungsprozeß ist darüberhinaus so langwierig - je nach Verbrennungsgrad einige Monate bis über ein Jahr -, daß der damit einhergehende Kostenaufwand sehr groß ist.

Aus diesem Grunde sind unter großem Forschungsaufwand Behandlungsverfahren entwickelt worden, bei denen der Bildung hypertropher Narben möglichst entgegengewirkt wird. Es liegen denn auch mehrere Varianten des eingangs genannten Behandlungsverfahrens vor.

So ist beispielsweise bekannt, um mittels Druckverbänden oder Mullkompressen beziehungsweise individuell anmodellierter, formfester Stützverbände oder Schienen Druck auf das Narbengewebe auszuüben. Es hat sich erwiesen, daß die Genesungsdauer einer Narbe durch Druckausübung verkürzt werden kann. Dieses bekannte Behandlungsverfahren hat jedoch einige Nachteile. So hat sich der durch Druckverbände ausgeübte Druck als unzureichend erwiesen, während formfeste Stützverbände, die in der Regel aus hartem Kunststoff hergestellt werden, die Hautgenesung beeinträchtigen.

Ferner ist zur Behandlung derartigen Narbengewebes ein Verfahren bekannt, bei dem kein Druck ausgeübt wird, sondern das Gewebe von der Umgebung abgeschrimt wird, indem darauf ein Abdeckkörper aus einem gewebeverträglichen und geschmeidigen Polymermaterial, vorzugsweise Silikon angebracht wird. Es hat sich herausgestellt, daß derartige Abdeckkörper die Hautgenesung fördern, wahrscheinlich weil auf diese Weise Flüssigkeitsverlusten entgegengewirkt wird. Da kein Druck ausgeübt wird, kann auf diese Weise jedoch der Bildung hypertrophen Narbengewebes nicht entgegengewirkt werden.

Schließlich sind auch Kombinationen dieser Behandlungsverfahren bekannt, bei denen Abdeckkörper aus Silikon zur Anwendung kommen, die fest auf das Narbengewebe gedrückt werden. Dieses Behandlungsverfahren vereint zwar die Vorteile beider genannter Verfahren in sich, doch in der Praxis erweist sich der auf diese Weise erzielte Druck als nicht optimal, wahrscheinlich weil die Kraft, mit der auf diese Weise auf das Narbengewebe zurückgedrängt wird, nicht während des gesamten Genesungsprozesses ausreichend ist.

Darüberhinaus haben alle obengenannten Behandlungsverfahren den Nachteil, daß das Narbengewebe während der Behandlung abgedeckt und unzugänglich ist, so daß darauf keine Wirkstoffe wie Heilmittel, die den Genesungsprozeß fördern könnten, aufgetragen werden können.

Die eingangs beschriebene Vorrichtung läßt sich der US-A-4,224,945 entnehmen. Diese Vorveröffentlichung offenbart einen Pflasterverband mit aufblasbarem Druckpolster. Das Pflaster besteht im wesentlichen aus einer unelastischen Folie, die ein Druckpolster abdeckt und mit einem freien Rand allseitig umschließt. Mit diesem freien Rand wird das Pflaster über einen Klebstoffilm auf die gesunde Haut des Patienten so geklebt, daß das Druckpolster mit einer Mullage auf dem zu behandelnden Geschwür bzw. der zu behandelnden Verletzung aufliegt. Das Druckpolster kann z. B. über eine Pumpe aufgeblasen werden. Unter Einwirkung dieses Aufblasdruckes wird die das Druckpolster von oben abdeckende unelastische Folie nach oben, also vom Geschwür weggedrückt, wodurch in dem vorstehend genannten Randbereich des Pflasters zentripetal gerichtete Zugkräfte auftreten, die über den Klebstoff auf die Haut des Patienten übertragen werden und dadurch die Haut in Richtung auf den Geschwürrand ziehen. Zugleich übt der Aufblasdruck auch einen nach innen über die Mulllage auf das Geschwür gerichteten Druck aus. Dabei kann zwischen dem Druckpolster und der genannten Mullage noch ein mit einem Wirkstoff getränkter Film vorgesehen sein.

Der Erfindung liegt die Aufgabe zugrunde, die eingangs beschriebene Vorrichtung hinsichtlich des zu fördernden Heilungsprozesses zu verbessern.

Ausgehend von der eingangs beschriebenen Vorrichtung wird diese Aufgabe erfindungsgemäß durch folgende Merkmale gelöst:
a) das Trageteil ist angenähert so groß ausgebildet wie der Abdeckkörper;
b) das Druckmedium zum Befüllen des zumindest einen Druckelementes enthält einen zur Behandlung des Narbengewebes bestimmten Wirkstoff;
c) die dem Trageteil abgewandte Seite des Abdeckkörpers bildet eine mit dem Narbengewebe in Berührung zu bringende Druckfläche, die aus einem selektiv permeablen Material besteht, das gegenüber dem im Druckmedium enthaltenen Wirkstoff durchlässig ist.

Hierdurch läßt sich der Druck während des Genesungsprozesses jeweils dem vom Narbengewebe ausgeübten Gegendruck anpassen, so daß die Bildung hypertropher Narben verhindert oder doch wenigstens verzögert werden kann. Zugleich kann dem Narbengewebe über den Abdeckkörper dosiert ein Wirkstoff zugeführt werden; trotz des Abdeckkörpers kann somit das Narbengewebe von außen gepflegt werden.

Weitere Merkmale der Erfindung sind Gegenstand der Unteransprüche und werden in Verbindung mit weiteren Vorteilen der Erfindung anhand von Ausführungsbeispielen näher erläutert.

Die Erfindung wird im folgenden anhand eines Beispiels beschrieben, wobei auf beiliegende Figuren verwiesen wird:
Figur 1 ist eine perspektivische Vorderansicht einer ersten Ausführung der Vorrichtung entsprechend der Erfindung;
Figur 2 ist eine Vorderansicht des Trageteils der in Figur 1 abgebildeten Vorrichtung;
Figur 3 ist eine Vorderansicht des Abdeckkörpers der in Figur 1 abgebildeten Vorrichtung;
Figur 4 zeigt die Druckregelvorrichtung der in Figur 1 abgebildeten Vorrichtung;
Figur 5 zeigt die Vorrichtung zur Befestigung der in Figur 1 abgebildeten Vorrichtung; und
Figur 6 zeigt eine andere Ausführung der Vorrichtung entsprechend der Erfindung.

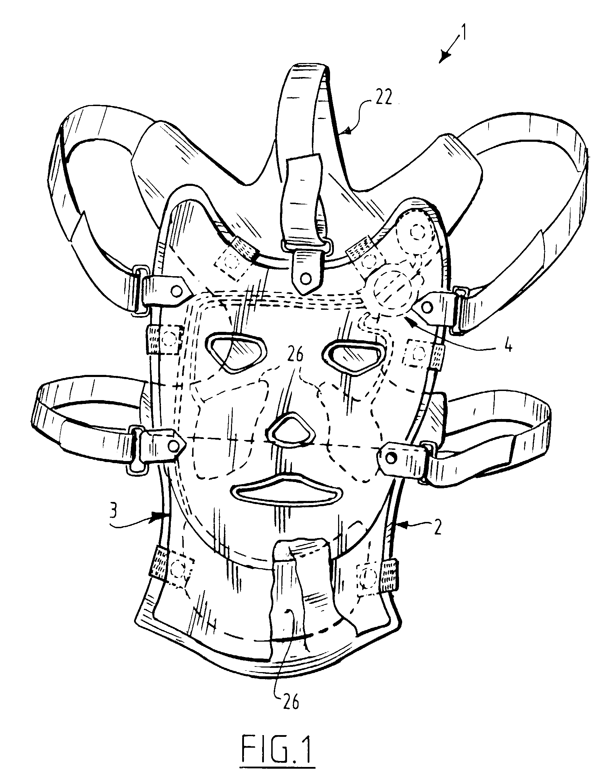

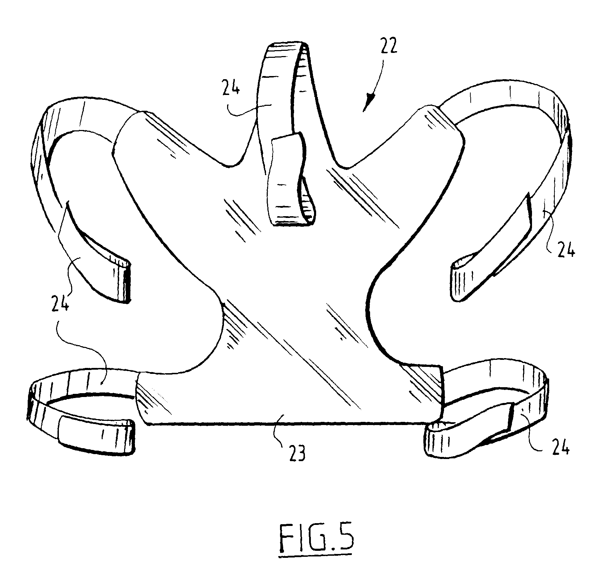

Eine Vorrichtung 1 zur Behandlung von Narbengewebe besteht aus einem mit dem Narbengewebe in Berührung zu bringenden Abdeckkörper 2 und einem damit verbundenen Trageteil 3, das den Abdeckkörper 2 auf das Gewebe drückt (Figur 1). Der Abdeckkörper 2 ist aus einem geschmeidigen, gewebeverträglichen Polymermaterial wie Silikon hergestellt, während das Trageteil 3 aus einem formfesten Material wie hartem Kunststoff oder Leichtmetall hergestellt ist. Form und Größe des Abdeckkörpers 2 und des Trageteils 3 sind denen des Körperteils, auf dem sich das Narbengewebe befindet angepast, wobei der Abdeckkörper 2 geringfügig über das Trageteil 3 hinausragt, um ein gewisses Maß an Komfort beim Tragen zu gewährleisten. Die Vorrichtung 1, die in vorliegendem Beispiel einer Maske zur Behandlung von Brandwunden im Gesicht entspricht, wird folglich im Prinzip für einen bestimmten Patienten maßgefertigr. Die Vorrichtung 1 ist ferner mit Mitteln 22 zur Befestigung des Trageteils 3 und des Abdeckkörpers 2 am zu behandelnden Körperteil versehen - in vorliegendem Beispiel in Form eines Kopfgestells mit einigen um den Kopf des Patienten anzubringenden Bändern 24 und einer diese Bänder 24 verbindenden Druckausgleichsplatte 23 (Figur 5). Bei einer ersten Ausführung der Erfindung befindet sich zwischen dem Trageteil 3 und dem Abdeckkörper 2 ferner Mittel 4 zur Regelung der Kraft, mit der der Abdeckkörper 2 auf das Gewebe gedrückt wird, welch Druckregelmittel 4 in den Abdeckkörper 2 integriert sind.

Das Trageteil 3 (Figuur 2) besteht in vorliegendem Beispiel aus einem das Gesicht umfassenden Teil 5 und einem damit verbundenen Halsteil 9. Im Gesichtsteil 5 befinden sich Öffnungen 6, 7 und 8 für die Augen, die Nase beziehungsweise den Mund des Patienten. Ferner besitzt das Trageteil Mittel 10 zur Verbindung davon mit dem Abdeckkörper 2, in vorliegendem Beispiel in Form von kleinen Klettbandteilen. Zur Befestigung am Kopfgestell 22 befinden sich am Rand des Trageteils 3 schließlich noch Befestigungsriemen 21.

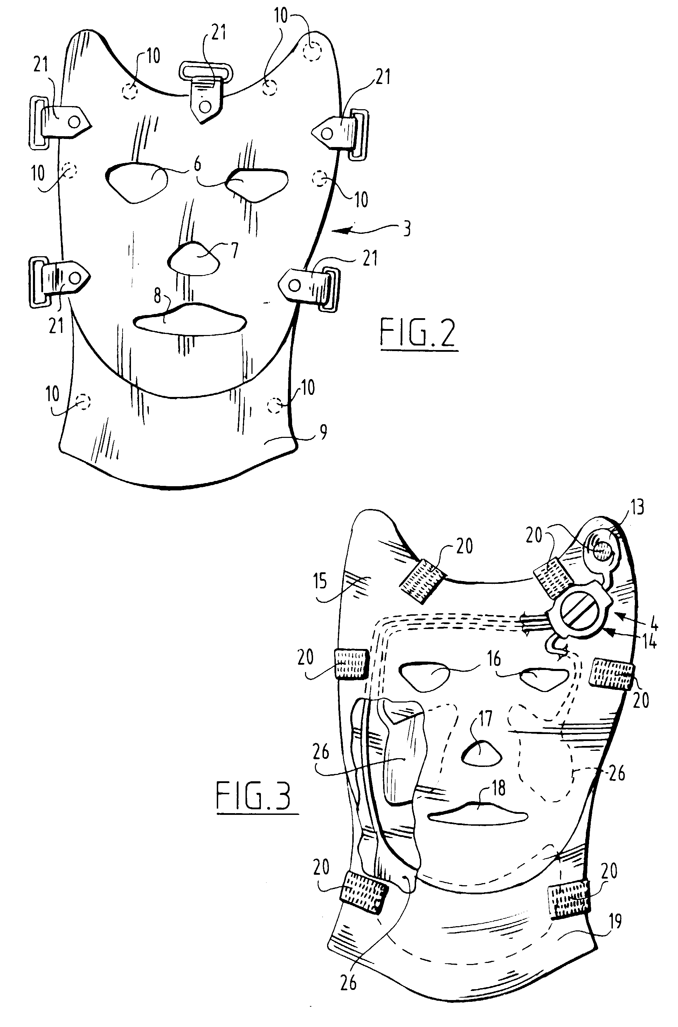

Der Abdeckkörper 2 (Figur 3), der hinsichtlich Form und Größe wie gesagt im wesentlichen dem Trageteil 3 entspricht, besteht ebenfalls aus einem Gesichtsteil 15 und einem Halsteil 19. Im Gesichtsteil 15 befinden sich wiederum Öffnungen 16, 17 und 18 für die Augen, die Nase und den Mund. Der Abdeckkörper 2 besitzt ferner mit den Verbindungsmitteln 10 des Trageteils 3 zusammenarbeitende Verbindungsmittel 20, ebenfalls in Form von kleinen Klettbandteilen. Außerdem ist der Abdeckkörper 2 mit den Mitteln 4 zur Regelung der Druckkraft desselben versehen. Die Druckregelmittel sind in vorliegendem Beispiel pneumatisch, und werden also von einem gasförmigen Medium, in vorliegendem Beispiel einfach Luft, bedient. Es wäre jedoch ebenfalls möglich, diese Mittel hydraulisch auszuführen, wobei sie also von einer Druckflüssigkeit bedient würden. Die Druckflüssigkeit könnte in diesem Fall mit vorteil gekühlt werden, wodurch zusätzlich die Wundschmerzen erleichtert würden. Auch völlig andere Formen der Druckkraftregelung, beispielsweise auf mechanischem Wege, kommen diesbezüglich in Betracht. Die Druckregelmittel 4 bestehen aus einigen aufblasbaren Elementen 26, die sich jeweils zwischen dem Trageteil 3 und dem Abdeckkörper 2 befinden, wodurch der Abdeckkörper 2 örtlich kräftig auf das Narbengewebe gedrückt wird. In vorliegendem Beispiel sind die aufblasbaren Elemente 26 in den Abdeckkörper 2 integriert, und sie bestehen aus einigen im Abdeckkörper 2 angebrachten Hohlräumen und damit verbundenen Leitungen 12. Wo sich diese aufblasbaren Elemente 26 genau befinden, hängt selbstverständlich von der genauen Stelle der zu behandelnden Narben ab und wird somit je Abdeckkörper 2 variieren.

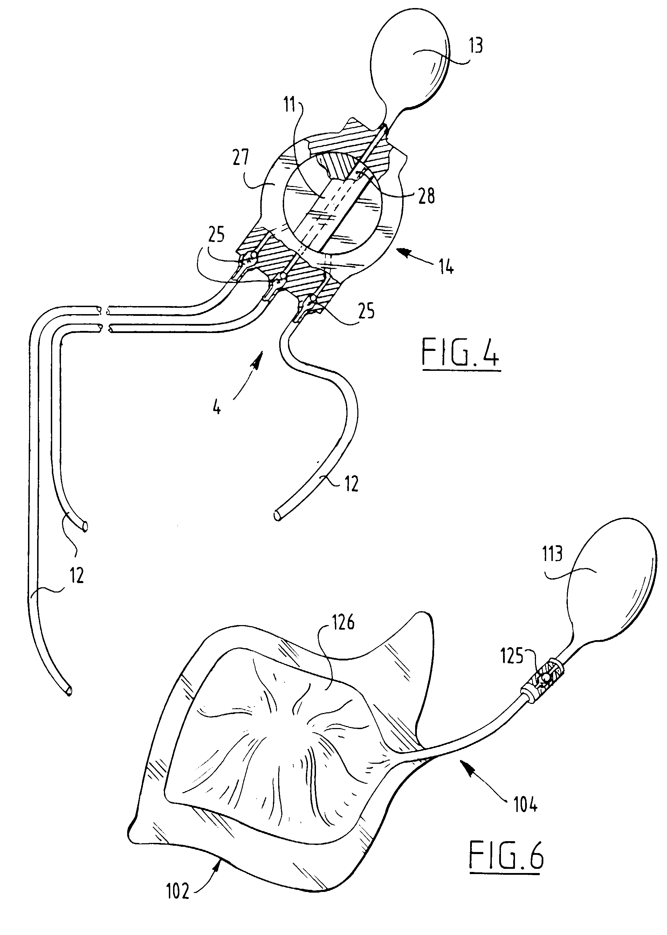

Die aufblasbaren Elemente 26 stehen über Rückschlagventile 25 (Figur 4), die verhindern, daß der Druck in den Elementen 26 nachläßt, mit regelbaren Pumpmitteln 13 in Verbindung, in vorliegendem Beispiel in Form einer einfachen Handpumpe. Obwohl im Prinzip selbstverständlich an jedes Element 26 eine Pumpe 13 angeschlossen werden könnte, befinden sich in vorliegendem Beispiel zwischen den Elementen 26 und der einzigen Pumpe 13 Schaltmittel 14, so daß die Pumpe 13 wahlweise jeweils mit einem der aufblasbaren Elemente 26 verbunden werden kann. Diese Schaltmittel 14 bestehen in vorliegendem Beispiel aus einem Dreiwegventil mit einem Ventilkörper 27, in dem ein sich darin befindlichen Schalter 11 mit darin integrierte Anschlußleitung 28 in drei Positionen gestellt werden kann, wobei dann die Pumpe 13 verbunden wird mit einer der Leitungen 12, die jeweils zu einem der aufblasbaren Elemente 26 führen.

Auf diese Weise läßt sich der Druck in jedem der aufblasbaren Elemente 26 abhängig vom Gegendruck des darunterliegenden Gewebes genau regeln. Darüber, wie hoch der Druck jeweils sein soll, gehen die Meinungen übrigens noch auseinander, wobei allgemein davon ausgegangen wird, daß dieser Druck auf jeden Fall höher sein sollte als der Kapillardruck im Narbengewebe, um der Bildung von Kollagenfasern zu verzögern oder sogar zu verhindern und eine Parallellagerung dieser Fasern zu bewirken, um auf diese Weise Zug zu verhindern. Auf jeden Fall können Gegendruckschwankungen, die während des Genesungsprozesses auftreten, ausgeglichen werden, so daß der Druck folglich unter allen Umständen einen optimalen Wert hat. Dies fördert die Genesung. So schätzungsweise läßt sich dem Genesungsprozeß sogar um etwa 30 Prozent verkürzen, was neben den Vorteilen für das Wohlbefinden des Patienten selbstverständlich ebenfalls mit erheblichen Kosteneinsparungen einhergeht. Im übrigen ist diese kürzere Genesungsdauer nicht nur eine Folge des unter allen Umständen optimalen Drucks, sondern es spielt dabei auch die Tatsache eine Rolle, daß durch die Regelung des Drucks ebenfalls eine optimale Paßform des Abdeckkörpers erreicht wird, da dieser gleichmäßig auf den verletzten Teil des Körpers gedrückt wird. Dies führt zu mehr Komfort beim Tragen und zu einem optimalen Kontakt zwischen dem Material des Abdeckkörpers und der Wunde.

Die oben beschriebene Ausführung der Vorrichtung entsprechend der Erfindung eignet sich insbesondere zur Behandlung großflächiger Narben, wie sie häufig im Fall einer Verbrennung der Gesichtshaut auftreten. Bei einer anderen Ausführung zur Behandlung von weniger ausgeprägten Narben (Figur 6) bildet der Abdeckkörper 102 zusammen mit einem aufblasbaren Element 126 ein Druckkissen, das über eine Leitung 112 und einen Rückschlagventil 125 mit einer Handpumpe 113 verbunden ist. Dieses Druckkissen kann beispielsweise mit einem (nicht abgebildeten) Stützverband, z. B. einer Schiene oder Druckverband auf dem zu behandelnden Narbengewebe fixiert werden.

Beide obengenannte Ausführungen der Vorrichtung können entsprechend einem zweiten Merkmal der Erfindung auf einfache Weise so ausgelegt werden, daß dem Narbengewebe über den Abdeckkörper 2 dosiert ein Wirkstoff zugeführt werden kann. Der Abdeckkörper muß dazu wirkstoffdurchlässig sein, was sich durch die Verwendung eines selektiv permeablen Materials für den Abdeckkörper erzielen läßt. Um den Wirkstoff dem Abdeckkörper zuzuführen, können dieselben Vorrichtungen wie zur Regelung der Druckkraft verwendet werden. Die Pumpmittenl 13, 113 können dazu durch einen Wirkstoffbehälter ersetzt werden. Auf diese Weise kann der Wirkstoff über die Leitungen 12, 112 zu den Elementen 26, 126 fließen, wo der Wirkstoff anschließend allmählich durch den Abdeckkörper 2 durchdringt und vom Narbengewebe aufgenommen wird. Das Narbengewebe kann auf diese Weise behandelt werden und dennoch von der Umgebungsluft abgeschirmt bleiben, wodurch der Genesungsprozeß ebenfalls erheblich verkürzt wird.

Selbstverständlich ist es ebenfalls möglich, beide Aspekte der Erfindung zu kombinieren, indem die Vorrichtung so ausgelegt wird, daß der Wirkstoff als Druckmedium verwendet wird und folglich ein konstant regelbarer Druck des Wirkstoffes erzielt wird. Es wird davon ausgegangen, daß sich die Genesung von Narbengewebe durch eine derartige Kombination von Behandlungsverfahren, die alle obengenannten Vorteile in sich vereint, erheblich fördern läßt, was das Wohlbefinden des Patienten steigert und darüber hinaus sowohl direkt als auch indirekt zu Kosteneinsparungen im Gesundheitswesen führen wird.

Obwohl die Erfindung oben anhand einiger spezifischer Anwendungsbeispiele beschrieben worden ist, sei darauf hingewiesen, daß sie selbstverständlich darauf nicht beschränkt ist.

## Patentansprüche

1. Vorrichtung zur Behandlung von Narbengewebe, zu dessen Abdeckung die Vorrichtung (1) einen Abdeckkörper (2) umfaßt, der zumindest ein mit einem Druckmedium befüllbares Druckelement (26) zur Druckbeaufschlagung des Narbengewebes aufweist und von einem dem Druckelement (26) als Widerlager dienenden Trageteil (3) übergriffen ist, das mit Befestigungsmitteln (22, 23, 24) am Körper des Patienten festlegbar ist, **gekennzeichnet durch** folgende Merkmale:
a) das Trageteil (3) ist angenähert so groß ausgebildet wie der Abdeckkörper (2);
b) das Druckmedium zum Befüllen des zumindest einen Druckelementes (26) enthält einen zur Behandlung des Narbengewebes bestimmten Wirkstoff;
c) die dem Trageteil (3) abgewandte Seite des Abdeckkörpers (2) bildet eine mit dem Narbengewebe in Berührung zu bringende Druckfläche, die aus einem selektiv permeablen Material besteht, das gegenüber dem im Druckmedium enthaltenen Wirkstoff durchlässig ist.

2. Vorrichtung nach Anspruch 1, **dadurch gekennzeichnet,** daß an das zumindest eine Druckelement (26) regelbare Pumpmittel (13, 113) angeschlossen sind.

3. Vorrichtung nach Anspruch 2, **dadurch gekennzeichnet,** daß der Abdeckkörper (2) mehrere, diskrete Druckelemente (26) aufweist.

4. Vorrichtung nach Anspruch 3, **dadurch gekennzeichnet,** daß zwischen den diskreten Druckelementen (26) und den Pumpmitteln (13, 113) Schaltmittel vorgesehen sind, über die die Pumpmittel (13, 113) wahlweise mit einem oder mehreren Druckelementen (26) verbindbar sind.

5. Vorrichtung nach einem der vorhergehenden Ansprüche, **dadurch gekennzeichnet,** daß die Druckfläche des Abdeckkörpers (2) aus einem gewebeverträglichen Polymermaterial besteht.

6. Vorrichtung nach einem der vorhergehenden Ansprüche, **dadurch gekennzeichnet,** daß der Abdeckkörper (2) trennbar mit dem Trageteil (3) verbunden ist.

7. Vorrichtung nach einem der vorhergehenden Ansprüche, **dadurch gekennzeichnet,** daß das Druckmedium zum Befüllen des oder der Druckelemente (26) gekühlt ist.

## Claims

1. Device for treating scar tissue, to cover which the device (1) includes a covering body (2), which comprises at least one pressure element (26), which can be filled with a pressure medium, for applying pressure to the scar tissue, and over which body a support part (3) engages, which part serves as an abutment for the pressure element (26) and can be fixed to the body of the patient with fastening means (22, 23, 24), characterised by the following features:
a) the support part (3) is approximately of the same size as the covering body (2);
b) the pressure medium for filling the pressure element (26), of which there is at least one, contains an active substance intended for treating the scar tissue;
c) the side of the covering body (2) which is distant from the support part (3) forms a pressure surface which is to be brought into contact with the scar tissue and consists of a selectively permeable material which is permeable to the active substance contained in the pressure medium.

2. Device according to claim 1, characterised in that controllable pumping means (13, 113) are connected to the pressure element (26), of which there is at least one.

3. Device according to claim 2, characterised in that the covering body (2) comprises a plurality of discrete pressure elements (26).

4. Device according to claim 3, characterised in that switching means are provided between the discrete pressure elements (26) and the pumping means (13, 113), via which means the pumping means (13, 113) can be connected either to one or to a plurality of pressure elements (26).

5. Device according to any one of the preceding claims, characterised in that the pressure surface of the covering body (2) consists of a polymer material which is compatible with tissue.

6. Device according to any one of the preceding claims, characterised in that the covering body (2) is connected to the support part (3) in a separable manner.

7. Device according to any one of the preceding claims, characterised in that the pressure medium for filling the pressure element(s) (26) is cooled.

## Revendications

1. Dispositif pour le traitement de tissu cicatriciel, le dispositif (1) comprenant pour recouvrir le tissu cicatriciel un corps de recouvrement (2) qui présente au moins un élément presseur (26) remplissable avec un médium sous pression pour appliquer une pression sur le tissu cicatriciel, le corps de recouvrement étant surmonté par une partie porteuse (3) qui sert de soutien à l'élément presseur (26) et peut être fixée au corps du patient par des moyens de fixation (22, 23, 24), caractérisé par les particularités suivantes:
a) la partie porteuse (3) est réalisée à peu près aussi grande que le corps de recouvrement (2) ;
b) le médium sous pression pour remplir l'au moins un élément presseur (26) contient une substance active définie pour le traitement du tissu cicatriciel;
c) le côté du corps de recouvrement (2) opposé à la partie porteuse (3) constitue une surface de pression à mettre en contact avec le tissu cicatriciel, qui est constituée d'un matériau à perméabilité sélective, qui est perméable à l'égard de la substance active contenue dans le médium sous pression.

2. Dispositif suivant la revendication 1, caractérisé en ce que des moyens de pompage (13, 113) pouvant être régulés sont raccordés à l'au moins un élément presseur (26).

3. Dispositif suivant la revendication 2, caractérisé en ce que le corps de recouvrement (2) présente plusieurs éléments presseurs discrets (26).

4. Dispositif suivant la revendication 3, caractérisé en ce qu'il est prévu, entre les éléments presseurs discrets (26) et les moyens de pompage (13, 113), des moyens de distribution par l'intermédiaire desquels les moyens de pompage (13, 113) peuvent être reliés au choix avec un ou plusieurs des éléments presseurs (26).

5. Dispositif suivant l'une des revendications précédentes, caractérisé en ce que la face de pression du corps de recouvrement (2) est constituée d'un matériau polymère compatible avec le tissu.

6. Dispositif suivant l'une des revendications précédentes, caractérisé en ce que le corps de recouvrement (2) est relié de manière séparable avec la partie porteuse (3) .

7. Dispositif suivant l'une des revendications précédentes, caractérisé en ce que le médium sous pression est refroidi pour le remplissage du ou des élément(s) de pression (26).
